# EUROPÄISCHE PATENTSCHRIFT

(11) **EP 1 461 139 B1**
(45) Veröffentlichungstag und Bekanntmachung des Hinweises auf die Patenterteilung: **31.08.2005**
(21) Anmeldenummer: 02787376.9
(22) Anmeldetag: 14.11.2002
(51) Int. Cl.: B01D 53/73, B01D 53/78, B01D 53/54, B01J 10/00, B01J 3/04, C07C 273/12, C07D 251/60

(54) **VERFAHREN ZUR REINIGUNG VON OFFGASEN EINER MELAMINANLAGE**
METHOD FOR PURIFYING OFF-GASES FROM A MELAMINE-PRODUCING INSTALLATION
PROCEDE DE PURIFICATION DE GAZ DE DEGAGEMENT PRODUITS PAR UNE INSTALLATION DE FABRICATION DE MELAMINE

(30) Priorität: 16.11.2001 AT 20011806; 25.06.2002 DE 10229101
(43) Veröffentlichungstag der Anmeldung: 29.09.2004
(73) Patentinhaber: AMI Agrolinz Melamine International GmbH, 4021 Linz (AT)
(72) Erfinder: SCHRÖDER, Frank, 04683 Albrechtshain (DE); BUCKA, Hartmut, A-4622 Eggendorf (AT); NEUMÜLLER, Christoph, A-4020 Linz (AT); COUFAL, Gerhard, A-4060 Leonding (AT)
(74) Vertreter: Gross, Felix
(86) Internationale Anmeldenummer: PCT/DE2002/004250
(87) Internationale Veröffentlichungsnummer: WO 2003/045538

(56) Entgegenhaltungen:
- EP-A- 0 459 962
- EP-A- 0 711 729
- JP-A- 53 081 898
- SU-A- 863 746
- SU-A- 1 074 161
- US-A- 3 700 672
- US-B1- 6 245 909

## Beschreibung

Die Erfindung betrifft ein zweistufiges Verfahren zur Reinigung von Offgasen aus einer Hochdruck-Melaminanlage mit melaminvorprodukt- und NH₃-hältiger Kreislaufharnstoffschmelze in einer ersten Stufe und mit frischer Harnstoffschmelze in einer zweiten Stufe.

Bei den Hochdruckverfahren zur Herstellung von Melamin werden im allgemeinen Harnstoff und Ammoniak bei Temperaturen zwischen etwa 320 und 450°C und Drücken zwischen etwa 50 und 600 bar zu flüssigem Melamin und Offgas, hauptsächlich bestehend aus Ammoniak, Kohlendioxid sowie geringen Mengen an gasförmigem Melamin und sonstigen Begleitprodukten, umgesetzt. Nach der Abtrennung der Melaminschmelze vom Offgas wird diese nach verschiedenen Verfahren zur Herstellung von reinem Melamin aufgearbeitet, während das Offgas vorzugsweise in eine Harnstoffanlage rückgeführt wird.
Vor der Rückführung des Offgases in die Harnstoffanlage muss dieses jedoch vom Melaminanteil und den sonstigen Begleitprodukten befreit werden, da diese in der Harnstoffanlage unerwünscht sind.
Gemäß US 3 700 672 wird das Offgas bei Temperaturen zwischen 135 und 250 °C und annähernd beim Synthesedruck des Melaminreaktors, das heißt bei 50 bis 200 bar mit Frischharnstoff im Gegenstrom einstufig in Kontakt gebracht und dabei vom vorhandenen Melamin und den sonstigen Begleitprodukten befreit. Das Melamin und die Begleitprodukte im Offgas werden in der Frischharnstoffschmelze absorbiert, somit aus dem Offgas rückgewonnen und anschließend erneut dem Melaminreaktor zugeführt. Dadurch kann die Melamin-Ausbeute im Melaminsynthesereaktor erhöht werden.
In dem gemäß US 3 700 672 angewandten Verfahren beträgt die maximale Betriebstemperatur für die Offgaswäsche 250 °C. Diese Temperaturobergrenze ist gemäß US 3 700 672 nötig, da oberhalb von 250 °C sowohl feste Nebenprodukte, die bei der Rezyklierung des Harnstoffes in den Melaminsynthesereaktor stören als auch gasförmige Nebenprodukte, die im Offgas unerwünscht sind, gebildet werden.

Der Nachteil des genannten Verfahrens ist, dass durch die einstufige Kontaktierung zwischen Offgas und Harnstoffschmelze die Austrittstemperaturen sowohl des gereinigten Offgases am Wäscherkopf als auch der mit Melamin und sonstigen Offgas-Begleitprödükten angereicherten Harnstoffschmelze im Wäschersumpf zwangsweise annähernd dieselben sind. Für die Einhaltung der geforderten niedrigen Betriebstemperaturen im Offgaswäscher ist es daher nötig, einen großen Teil der mit dem heissen Offgas in den Wäscher eingetragenen Wärme über einen Kühler als Abwärme abzuführen. Diese Abwärme kann zwar zur Bildung von Dampf verwendet werden, in der Energiebilanz des Melaminsyntheseprozesses bedeutet sie jedoch einen Energieverlust, da die aus dem Wäscher mit maximal 250 °C ausgetragene Harnstoffschmelze im Melaminreaktor erneut auf die Synthesetemperatur von etwa 380 °C aufgewärmt werden muss. Dies bedeutet, dass der Energieverlust im Wäscher durch die Zufuhr von Heizenergie im Synthesereaktor ausgeglichen werden muss.
Ein weiterer Nachteil niedriger Betriebstemperaturen im Wäschersumpf besteht in der Bildung von Nebenprodukten. Dies sind solche Stoffe, die sich im Wäscher exotherm, das heißt unter Wärmeabgabe aus dem Ammoniak und dem Kohlendioxid des Offgases bilden, wie beispielsweise Carbamat und Wasser. Durch Bildung dieser Stoffe entsteht im Wäscher Wärme auf niedrigem Temperatumiveau, welche als Abwärme abgeführt werden muss. In weiterer Folge müssen diese Nebenprodukte im Melaminreaktor durch Zufuhr von Heizenergie auf hohem Temperatumiveau erneut in die Ausgangsstoffe Ammoniak und Kohlendioxid zersetzt werden. Dieser Energietransport in Form von chemischer Energie vom Synthesereaktor zum Wäscher ist ein großer Verlust.

Die Anforderungen an einen optimalen Offgaswäscher sind demnach vielfältig: einerseits sollen die vom Melaminreaktor kommenden Offgase möglichst vollständig vom Melamin und den weiteren Begleitstoffen befreit werden, andererseits soll die Energieeffizienz des Melaminsyntheseprozesses durch bessere Nutzung der Offgaswärme verbessert werden.

Es stellte sich demnach die Aufgabe, ein Verfahren zu finden, das diesen Anforderungen gerecht wird.

Unerwarteterweise konnte ein Verfahren entwickelt werden, mit welchem sowohl die Offgase vor deren Rückführung in die Harnstoffanlage effizient gereinigt werden als auch durch Umwandlung von Wärmeenergie der Offgase in chemische Energie von Melaminvorprodukten im Wäscher die Energieeffizienz des Melaminprozesses gesteigert werden kann.

Gegenstand der vorliegenden Erfindung ist demnach ein Verfahren zur Reinigung von Offgasen aus einer Hochdruck-Melaminanlage unter Bildung von Melaminvorprodukten, das dadurch gekennzeichnet ist, dass die Offgase in einer ersten Stufe mit melaminvorprodukt- und NH₃-hältiger Kreislaufharnstoffschmelze und in einer zweiten Stufe mit frischer Harnstoffschmelze in Kontakt gebracht werden.

Die erfindungsgemäße Offgasreinigung bietet zahlreiche Vorteile, die im folgenden erläutert werden:
Die Offgasreinigung in einer ersten, heißeren Stufe mit melaminvorprodukt- und NH₃-hältiger Kreislaufharnstoffschmelze und in einer zweiten, kälteren Stufe mit frischer Harnstoffschmelze verbessert im Vergleich zu einstufiger Kontaktierung bei konstanter Temperatur den Abscheidegrad der in den Offgasen vorhandenen Begleitprodukte sodaß bei sonst gleichen Betriebsbedingungen am Kopf des zweistufigen Offgaswäschers reinere Offgase im Vergleich zum einstufigen Wäscher abgezogen werden können. Weiters ist durch die in der ersten Stufe mit melaminvorprodukt- und NH₃-hältiger Kreislaufharnstoffschmelze erreichte Vorwäsche bereits am Übergang der Offgase von der ersten in die zweite Stufe der Feststoffgehalt in den Offgasen soweit erniedrigt, daß der Einsatz von hochwirksamen Waschelementen wie beispielsweise Sieb- oder Ventilböden zur Feinabscheidung am Wäscherkopf möglich ist, ohne dass es zur Verlegung beziehungsweise Verstopfung dieser Elemente kommt, wodurch sich wiederum die Effizienz der Offgasreinigung verbessert.

Durch die erfindungsgemäße Offgasreinigung ist es zudem möglich, den Wäschersumpf bei einer anderen, nämlich einer höheren Temperatur als den Wäscherkopf zu betreiben. Dadurch kann der Harnstoff im Wäschersumpf gegenüber einem einstufigen Verfahren, bei welchem notwendigerweise Offgas- und Harnstoffaustrittstemperatur annähernd gleich sind, stärker vorgewärmt werden, was eine Verbesserung der Energiebilanz des Melaminsynthesereaktor-Offgaswäscher-Systems bedeutet.
Darüber hinaus wird es auf diese Weise möglich, die mit den Offgasen eingetragene Wärme teilweise in chemische Energie von Melaminvorprodukten umzuwandeln anstatt sie als nutzlose Abwärme über einen Kühler abzuführen. Unter Melaminvorprodukten werden solche Stoffe verstanden, die sich im Offgaswäscher unter bestimmten Bedingungen endotherm, das heißt unter Wärmeverbrauch aus dem Edukt Harnstoff bilden wie beispielsweise Ammelin, Ammelid oder Cyanursäure. Diese Stoffe werden anschließend gemeinsam mit der im Wäscher vorgewärmten Harnstoffschmelze dem Melaminsynthesereaktor zugeführt und dort in einer endothermen Reaktion zu Melamin umgesetzt. Da jedoch zur Melaminbildung ausgehend von den Melaminvorprodukten weniger Energie nötig ist als zur Melaminbildung ausgehend von Harnstoff kann auf diese Weise die im Melaminsynthesereaktor nötige Heizenergie emiedrigt werden. Durch Zufuhr von Kohlendioxid am Übergang der Offgase in die melaminvorprodukt-und NH₃-hältige Kreislaufharnstoffschmelze der ersten Stufe lässt sich die Melaminvorproduktbildung noch zusätzlich steigern.

Ein zusätzlicher Vorteil der höheren Betriebstemperatur in der ersten Stufe liegt darin, dass weniger Carbamat beziehungsweise Wasser aus dem Ammoniak und Kohlendioxid der Offgase gebildet werden.

### Die Erfindung wird im folgenden detailliert beschrieben:

Die zu reinigenden Offgase stammen aus einer beliebigen Hochdruck-Melaminanlage, beispielsweise stammen sie aus einem Melaminsynthesereaktor, einem Stripper, einem Aging-Behälter oder einem Schmelzekühler oder aus mehreren dieser Apparate oder Rohrleitungen. Die Offgase bestehen hauptsächlich aus Ammoniak und Kohlendioxid mit geringen Mengen an Melamin sowie gegebenenfalls weiteren Begleitstoffen wie beispielsweise Harnstoff, Ureidomelamin oder Melam.
Die Offgase werden mit einer Temperatur, die zwischen dem druckabhängigen Schmelzpunkt des Melamins und etwa 500 °C, bevorzugt zwischen etwa 360 und 400 °C liegt und einem Druck von etwa 50 bis 600 bar, bevorzugt von etwa 70 bis 400 bar in den Offgaswäscher eingetragen.

Der Offgaswäscher wird bevorzugt bei annähernd demselben Druck wie der Apparat, aus welchem die zu reinigenden Offgase stammen, betrieben, besonders bevorzugt wird er bei annähernd demselben Druck wie der Melaminsynthesereaktor betrieben.

Stammen die Offgase aus mehreren Apparaten der Hochdruck-Melaminanlage, so entspricht der Betriebsdruck des Offgaswäschers annähernd dem Druck desjenigen Apparates, der beim niedrigeren Druck betrieben wird.

Die Temperatur in der ersten Stufe des Offgaswäschers ist höher als die Temperatur in der zweiten Stufe. Dabei beträgt die Temperatur in der ersten Stufe etwa 160 bis 320 °C, bevorzugt etwa 180 bis 280 °C. Die Temperatur in der zweiten Stufe beträgt etwa 135 bis 300 °C, bevorzugt etwa 150 bis 270°C. Weiters ist es möglich, gemeinsam mit den Offgasen gasförmiges Kohlendioxid in den Offgaswäscher einzutragen. Dies hat den Vorteil, dass dadurch die Bildung von Melaminvorprodukten im Offgaswäscher gefördert wird.
Die Offgase und gegebenenfalls Kohlendioxid werden bevorzugt in fein verteilter Form in die erste Stufe des Offgaswäschers eingebracht, dies kann beispielsweise über einen Offgaskanal in einem Offgasverteiler erfolgen. Die erste Stufe reicht bevorzugt vom Wäschersumpf bis zu etwa zwei Drittel der Apparatehöhe.
Der Füllstand des Harnstoffwäschers mit Harnstoffschmelze ist variabel. Beispielsweise reicht er etwa bis zu einem Viertel der Apparatehöhe oder höher, wobei es auch möglich ist, den Apparat mit Ausnahme eines geringen Bereiches zur Offgasseparation am Apparatekopf praktisch geflutet zu betreiben.
Die Einbringung der Offgase in die erste Stufe kann entweder unterhalb des Flüssigkeitsspiegels der ersten Stufe oder oberhalb des Flüssigkeitsspiegels in den Gasraum erfolgen. Wenn der Offgaseintritt unter dem Flüssigkeitsspiegel erfolgt, wird die Offgasleitung bevorzugt im Verteiler von außen beheizt, um ein Kondensieren der Offgase an der Verteilerwand zu vermeiden. Um eine Überhitzung der umgebenden melaminvorprodukt- und NH₃-hältigen Kreislaufharnstoffschmelze zu verhindern, wird diese Heizung nach außen isoliert. Dazu eignet sich beispielsweise ein gasgespülter Ringraum, welcher an der Eintrittstelle der Offgase in die Schmelze durch Düsen an der tiefsten Stelle des Gasraumes mit der Schmelze verbunden ist. Im Falle von Kohlendioxidzugabe zu den eintretenden Offgasen kann dessen Zumischung zweckmäßigerweise im Verteiler erfolgen, und das Kohlendioxid kann neben der Förderung der Melaminvorproduktbildung auch als Spülgas dienen. Wird kein Kohlendioxid zugegeben, kann als Spülgas beispielsweise auch Ammoniak oder ein beliebiges Inertgas verwendet werden. Die Temperatur des zugeführten Kohlendioxid, Ammoniak oder Inertgases sollte vorzugsweise zwischen der Offgas- und der Harnstoffschmelzetemperatur der ersten Stufe liegen.
In der ersten Stufe des Offgaswäschers kontaktieren die eingebrachten Offgase, gegebenenfalls mit Kohlendioxid oder einem Spülgas vermischt, im Gegenstrom die melaminvorprodukt- und NH₃-hältige Kreislaufharnstoffschmelze. Die Kontaktierung kann auch im Kreuzstrom erfolgen. Als Kreislaufharnstoffschmelze wird eine Mischung von Harnstoffschmelze aus der zweiten Stufe und von über den Kreislauf in die erste Stufe rückgeführte Harnstoffschmelze verstanden. Die Kreislaufführung der Harnstoffschmelze in der ersten Stufe des Offgaswäschers ermöglicht einen besonders intensiven Gas-Flüssigkeitskontakt. Die melaminvorprodukt- und NH₃-hältige Kreislaufhamstoffschmelze wird am Sumpf des Harnstoffwäschers abgezogen und in zwei Teile aufgeteilt. Der erste Teil wird dem Offgaswäscher am oberen Ende der ersten Stufe in den Gasraum über dem Harnstoffschmelzespiegel bevorzugt in fein verteilter Form erneut zugeführt, der zweite, ausgekreiste Teil wird dem Melaminsynthesereaktor zugeführt.
Die Harnstoff-Kreislaufströmung in der ersten Stufe und der Transport der melaminvorprodukt- und NH₃-hältigen Kreislaufharnstoffschmelze zum Melaminsynthesereaktor können beispielsweise durch die Verwendung einer Pumpe oder durch Ausnutzung des Dichteunterschiedes zwischen der blasenfreien Harnstoff-Kreislaufströmung und der Zweiphasenströmung im Wäschersumpf nach dem Mammutpumpenprinzip realisiert werden. Die Kreislaufmenge ist bevorzugt ein Vielfaches der Frischhamstoffmenge.
Zur Energieabfuhr wird bevorzugt ein Wärmetauscher in den Kreislauf geschaltet. Er kann sowohl als interner Wärmetauscher im Offgaswäscher als auch als externer Wärmetauscher ausgeführt sein. Bevorzugt wird ein externer Wärmetauscher verwendet. Die am Wärmetauscher abgeführte Wärme kann gegebenenfalls zur Erzeugung von Dampf verwendet werden. Die Temperaturregelung in der ersten Stufe des Offgaswäschers erfolgt über die Energieabfuhr am Wärmetauscher, die Temperatur in der zweiten Stufe wird indirekt mitbeeinflusst.
Durch den intensiven Kontakt zwischen der melaminvorprodukt- und NH₃-hältigen Kreislaufharnstoffschmelze und den eingebrachten Offgasen kommt es zu einem Energie- und Stoffübergang von den Offgasen auf die Harnstoffschmelze der ersten Stufe. Der Energieübergang erfolgt in Form von Wärmeabgabe der Offgase auf die Harnstoffschmelze, welche dadurch vorgewärmt wird. Ein Teil dieser Wärme wird in chemische Energie unter Bildung von Melaminvorprodukten wie beispielsweise Ammelin, Ammelid oder Cyanursäure umgewandelt. Die Umwandlung von Wärmeenergie der Offgase in chemische Energie der Melaminvorprodukte ist wünschenswert, da auf diese Weise die über den Wärmetauscher des Harnstoff-Kreislaufes abzuführende Wärme, das heißt die Wärmeverluste im Melaminsynthesereaktor-Offgaswäscher-System verringert werden. Das Ausmaß der Melaminvorproduktbildung ist umso größer, je höher die Temperatur in der ersten Stufe des Offgaswäschers ist, weiters kann auch durch die gleichzeitige Einbringung von gasförmigem Kohlendioxid mit den Offgasen die Vorproduktbildung gesteigert werden. Das Ausmaß der Melaminvorproduktbildung im Offgaswäscher ist durch die Viskositätserhöhung und eventuell daraus resultierender Transportprobleme der melaminvorprodukt- und NH₃-hältigen Kreislaufharnstoffschmelze zum Melaminsynthesereaktor beziehungsweise im Harnstoff-Kreislauf mit steigender Temperatur begrenzt beziehungsweise je nach Anlagengestaltung variabel. Die Einstellung der für die gewünschte Melaminvorproduktbildung in der Harnstoffschmelze nötige Verweilzeit erfolgt durch die Gestaltung des Harnstoff-Kreislaufes der ersten Stufe.
Der Stoffübergang zwischen den Offgasen und der Harnstoffschmelze erfolgt in Form der Entfernung eines Großteils des Melamins und der restlichen Begleitstoffe wie beispielsweise Ureidomelamin oder Melam aus den Offgasen in der ersten Stufe des Offgaswäschers. Aufgrund deren niedriger Kristallisationstemperaturen werden diese Stoffe in der kühleren Kreislaufharnstoffschmelze absorbiert. Infolge der relativ hohen Temperatur und des in der Kreislaufharnstoffschmelze der ersten Stufe enthaltenen Ammoniaks können auch diese von den Offgasen abgetrennten Begleitstoffe gemeinsam mit dem Harnstoff teilweise in Melaminvorprodukte umgesetzt werden.
Je nach den Druck- und Temperaturbedingungen im Offgaswäscher können in der Harnstoffschmelze der ersten Stufe außerdem geringe Mengen an Nebenprodukten wie beispielsweise Carbamat und Wasser enthalten sein. Sie werden durch Kondensation von Ammoniak und Kohlendioxid der Offgase gebildet, wobei das Ausmaß der Nebenproduktbildung umso höher ist, je niedriger die Temperatur im Offgaswäscher ist. Die aus der ersten Wäscherstufe dem Melaminsynthesereaktor zugeführte melaminvorprodukt- und NH₃-hältige Kreislaufharnstoffschmelze hat eine Temperatur von etwa 160 bis 320 °C, sie enthält neben den von den Offgasen abgetrennten Begleitstoffen einen bestimmten Anteil an Melaminvorprodukten sowie gegebenenfalls geringe Mengen an Nebenprodukten und ist bevorzugt ammoniakgesättigt. Durch den Ammoniakgehalt der Kreislaufharnstoffschmelze aus dem Offgaswäscher ist es im allgemeinen nicht nötig, dem Melaminsynthesereaktor zusätzliches Ammoniak zuzuführen.

Die durch die Kreislaufharnstoffschmelze der ersten Stufe aufsteigenden Offgase treten oberhalb der Einsprühvorrichtung der Kreislaufharnstoffschmelze in die zweite Stufe ein. Gegebenenfalls kann in der zweiten Stufe, beispielsweise an deren unterem Ende eine Vorrichtung zur Feinabscheidung der in den Offgasen noch vorhandenen Begleitstoffe angebracht sein. Es ist auch möglich, mehrere Abscheidevorrichtungen zu verwenden, beispielsweise können ein oder mehrere Sieb- oder Ventilböden verwendet werden.

In der zweiten Stufe des Offgaswäschers kontaktieren die Offgase im Gegenstrom die am Kopf des Wäschers zugeführte frische Harnstoffschmelze, welche direkt aus einer Harnstoffanlage kommen kann. Die Kontaktierung kann auch im Kreuzstrom erfolgen. Die frische Harnstoffschmelze wird mit einer Temperatur von etwa 135 bis 180 °C, bevorzugt in feinverteilter Form, beispielsweise über Sprühdüsen, zugeführt. Durch den intensiven Kontakt zwischen den aufsteigenden heissen Offgasen und dem kalten frischen Harnstoff erfolgt die Endabscheidung nahezu aller noch in den Offgasen enthaltenen Begleitstoffe. Darüberhinaus erfolgt hier die Kühlung der Offgase auf die gewünschte Endtemperatur von etwa 170 bis 250 °C.
Die gereinigten Offgase, hauptsächlich bestehend aus Ammoniak und Kohlendioxid, werden am Kopf des Offgaswäschers abgezogen und anschließend bevorzugt in eine Harnstoffanlage rückgeführt.
Es ist auch möglich, die in der zweiten Stufe zugeführte frische Harnstoffschmelze auf mehrere Teilströme aufzuteilen und die einzelnen Teilströme beispielsweise in unterschiedlicher Höhe in die zweite Stufe des Offgaswäschers einzubringen.

Durch die Effizienz der erfindungsgemäßen Offgasreinigung ist es auch möglich, nur einen Teil der dem Melaminsynthesereaktor insgesamt zugeführten Harnstoffschmelze für die Offgasreinigung zu verwenden und den zweiten Teil der Harnstoffschmelze beispielsweise als frische Harnstoffschmelze direkt aus der Harnstoffanlage in den Melaminsynthesereaktor einzubringen. Dadurch kann die Menge der in den Melaminsynthesereaktor eingetragenen Nebenprodukte weiter verringert werden während gleichzeitig im erfindungsgemäßen Offgaswäscher die erwünschten Melaminvorprodukte gebildet werden.

Als Offgaswäscher kann ein beliebiger zweistufiger Apparat, wie beispielsweise für Staubabscheidung oder Absorption gebräuchlich, verwendet werden. Beispielsweise können Sprühturme, gepackte Säulen, Blasensäulen, Bodenkolonnen, Rieselfilmkolonnen oder Sedimentationskolonnen für eine, mehrere oder alle Wäscherstufen verwendet werden.

Bevorzugt ist der Offgaswäscher mit einer Heizvorrichtung, beispielsweise mit einer Dampfmantelheizung, versehen. Als Wärmetauscher wird beispielsweise ein Rohrbündelwärmetauscher verwendet.

Eine bevorzugte Ausführungsform der vorliegenden Erfindung wird nun anhand der beigefügten Zeichnung erklärt:

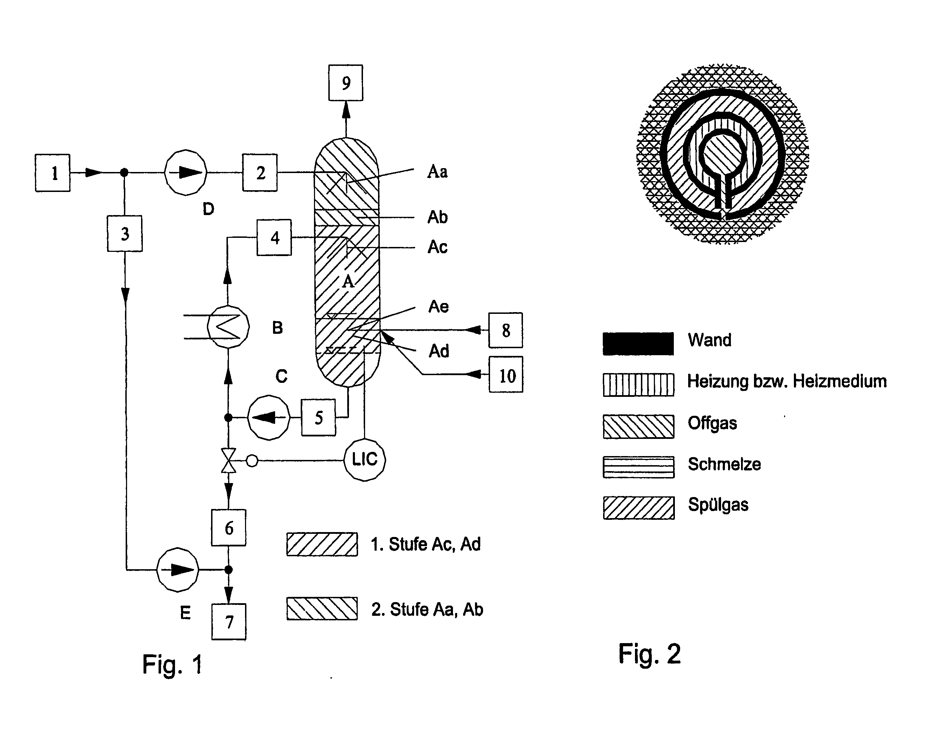

Fig. 1 zeigt ein Beispiel eines zweistufigen Offgaswäschers A entsprechend der vorliegenden Erfindung.
Die untere erste Stufe des Wäschers besteht in ihrem oberen Abschnitt aus einem unteren Sprühturm Ac, in welchem die melaminvorprodukt- und NH₃-hältige Harnstoffschmelze aus dem Kreislauf zugeführt wird. Im unteren Abschnitt der ersten Stufe befindet sich der Offgasverteiler Ae, welcher entweder oberhalb oder
unterhalb des Füllstandes der melaminvorprodukt- und NH₃-hältigen Kreistaufharnstoffschmelze in den Offgaswäscher einmündet. Befindet sich der Offgasverteiler unterhalb des Füllstandes, so ergibt sich ein zusätzlicher Blasensäulenabschnitt Ad am Apparatesumpf der ersten Stufe.
Die obere zweite Stufe des Offgaswäschers ist in einen oberen Sprühturm Aa und einen oder mehrere Siebböden Ab zur intensiven Wäsche der aus der ersten Stufe aufsteigenden Offgase untergliedert. In der zweiten Stufe erfolgt bei relativ niedriger Temperatur die Offgasreinigung mit frischer Harnstoffschmelze.
Der Frischharnstoffzulaufstrom 1 teilt sich in den Wäscher-Frischharnstoffzulaufstrom 2, der am Kopf über einen Verteiler in den Offgaswäscher strömt und in den Bypass-Frischharnstoffstrom 3, der direkt in den Melaminsynthesereaktor führt. Dabei kann der dem Offgaswäscher bygepaßte Frischharnstoffstrom 3 entweder dem Harnstoffschmelze-Auskreisungsstrom 6 aus dem Offgaswäscher vor dem Eintritt in den Melaminsynthesereaktor zugemischt und als Melaminsynthesezulaufstrom 7 oder als separater Harnstoffschmelze-Strom in den Melaminsynthesereaktor geführt werden.
Die Mengenaufteilung des Frischharnstoffzulaufstromes 1 erfolgt über die regelbare Frischharnstoffzulaufpumpe D und die regelbare Bypass-Frischharnstoffpumpe E. Mittels der Kreislaufharnstoffpumpe C wird am Sumpf der ersten Stufe des Offgaswäschers ein melaminvorprodukt- und NH₃-hältiger Kreislaufharnstoffstrom 5 abgezogen, welcher in den Wäscher-Kreislaufharnstoffstrom 4 und den Harnstoffschmelze-Auskreisungsstrom 6 aufgeteilt wird. Der Wäscher-Kreislaufharnstoffstrom 4 wird über den Wärmetauscher B mittels eines Verteilers in die erste Stufe rückgeführt. Der Harnstoffschmelze-Auskreisungsstrom 6, welcher über die Füllstandregelung LIC den Füllstand im Offgaswäscher regelt, wird nach Vermischen mit dem Bypass-Frischhamstoffstrom 3 als Melaminsynthesezulaufstrom 7 dem Melaminsynthesereaktor zugeführt.
Die gereinigten Offgase verlassen den Offgaswäscher am Kopf über den Offgasaustritt 9 in Richtung Harnstoffanlage.
Die aus der Hochdruck-Melaminanlage kommenden heißen Offgase werden am Offgaseintritt 8 im unteren Teil des Offgaswäschers, unterhalb des Verteilers für den Wäscher-Kreislaufharnstoffstrom 4, über den Offgasverteiler Ae in den Wäscher eingebracht. Gleichzeitig mit den Offgasen wird dem Offgaswäscher über die Kohlendioxidzugabe 10 Kohlendioxid zugeführt.
Fig. 2 zeigt einen Schnitt durch den Offgasverteiler Ae.
Er besteht aus einem von außen beheizten Offgaszentralrohr mit nach unten gerichteten Gasaustrittskanälen. Der Heizmantel des Offgaszentralrohrs ist mittels eines wärmeisolierenden, gasgespülten Ringraumes von der umgebenden Harnstoffschmelze getrennt. Der Ringraum ist durch Düsenbohrungen an der tiefsten Stelle mit dem Gasaustritt des Offgaskanals verbunden.
Auf diese Weise wird die Wärmeabgabe des Heizmediums an die Harnstoffschmelze möglichst gering gehalten, um die bei höheren Temperaturen ablaufende Pyrolyse des Harnstoffes, die zur Bildung von Pyrolyseprodukten führen würde, weitgehend zu vermeiden.
Die folgende Tabelle zeigt den Vergleich zwischen dem einstufigen und dem erfindungsgemäßen zweistufigen Verfahren zur Offgasreinigung.

| | einstufiges Verfahren | | zweistufiges Verfahren | |
|---|---|---|---|---|
| Temperatur | Offgas | Harnstoff | Offgas | Harnstoff |
| Eintritt | 370 °C | 150 °C | 370 °C | 150 °C |
| Übergang erste/zweite Stufe | - | - | 242 °C | 205 °C |
| Austritt | 205 °C | 205 °C | 205 °C | 242 °C |
| Spezifische Abwärme | 33 kJ/mol Harnstoff | | 24 kJ/mol Harnstoff | |
| Gehalt der dem Melaminsynthesereaktor zugeführten Harnstoffschmelze an Melaminvorprodukten und kondensiertem Melamin | 4 % | | 7 % | |

Bei gleicher Offgaseintritts-, Frischharnstoffeintritts-, Offgasaustrittstemperatur sowie gleichem Druck in beiden Wäschervarianten ergeben sich für den zweistufigen Offgaswäscher mit unterschiedlicher Betriebstemperatur in beiden Stufen bei gleicher Effizienz der Offgasreinigung eine bessere Harnstoffvorwärmung, geringere spezifische Abwärmeverluste sowie eine erhöhte Melaminvorproduktbildung was insgesamt einer verbesserten Energieeffizienz des Gesamtprozesses entspricht.
Fig. 3

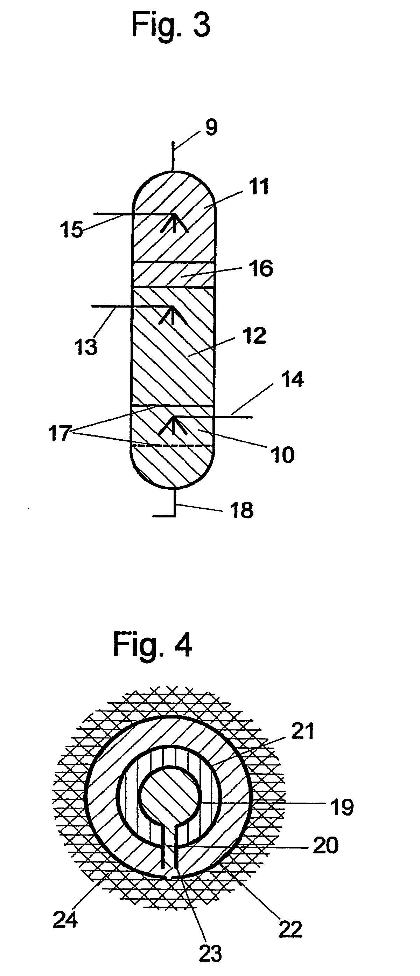

Fig. 3 zeigt einen schematischen Schnitt eines Gaswäschers wie er für die Reinigung vereunreinigter Gase benutzt wird. Vorteilhaft wird der Gaswäscher als Offgaswäscher zur Durchführung des erfindungsgemäßen Verfahrens benutzt.
Der Gaswäscher besteht aus zwei Stufen, die übereinander angeordnet sind. Im oberen Abschnitt der unteren Stufe (12) befindet sich ein Sprühturm (13), durch den eine Schmelze zugeführt wird. Im unteren Teil der unteren Stufe befindet sich der Gasverteiler, durch den das zu reinigende Gas eingeleitet wird. Beim erfindungsgemäßen Verfahren werden durch den Gasverteiler die zu reinigenden Offgase eingeleitet.
Im oberen Teil der oberen Stufe (11) ist ein weiterer Sprühturm (15) angeordnet durch den die Schmelze, bei der Erfindung Harnstoffschmelze, zugeführt wird. Dem Sprühturm (15) gegenüber befinden sich ein oder mehrere Siebböden zur intensiven Wäsche der aus der unteren Stufe aufsteigenden Gase. Die greinigten Gase werden am Kopf des Gaswäschers und die Schmelze am Sumpf der unteren Stufe entnommen.
Bei einer bevorzugten Ausführungsform des Gaswäschers ist es möglich, neben dem Gasverteiler im unteren Abschnitt der unteren Stufe eine oder mehrere weitere Gaszuführungen vorzusehen.
Es liegt auch im Rahmen der Erfindung, Gaswäscher mit einem Heizmantel auszustatten.
Fig. 4
Fig. 4 zeigt den Schnitt durch den Gasverteiler
Der Gasverteiler besteht aus einem Gaszentralrohr (19), das von einem Heizmantel (21) umgeben ist. Durch diese Ausgestaltung wird ein Wärmeverlust des zu reinigenden Gase weitgehend vermieden. Der Heizmantel (21) wird von einem thermischen Isoliermittel, vorzugsweise von einem gasgespülten Ringraum zur Isolierung des Heizmantels (21) von der Schmelze umgeben. Durch Düsenbohrungen kann das Gas aus dem Gasverteiler in die Schmelze austreten.

Die Düsenbohrungen können als Gasaustrittskanal durch zwei parallele Blechstreifen, die an beiden Schlitzkanten senkrecht an das Gaszentralrohr (19) angeschweißt sind, gebildet werden.

## Patentansprüche

1. Verfahren zur Reinigung von Offgasen aus einer Hochdruck-Melaminanlage unter Bildung von Melaminvorprodukten, **dadurch gekennzeichnet, dass**
- die Offgase in einer ersten Stufe mit melaminvorprodukt- und NH₃-hältiger
Kreislaufharnstoffschmelze und
- in einer zweiten Stufe mit frischer Harnstoffschmelze in Kontakt gebracht werden.

2. Verfahren nach Anspruch 1, **dadurch gekennzeichnet, dass** die melaminvorprodukt- und NH₃-hältige Kreislaufhamstoffschmelze eine Mischung von Harnstoffschmelze aus der zweiten Stufe und von über den Kreislauf in die erste Stufe rückgeführte Harnstoffschmelze ist.

3. Verfahren nach Anspruch 1 oder 2, **dadurch gekennzeichnet, dass** ein Teil der melaminvorprodukt- und NH₃-hältigen Kreislaufharnstoffschmelze dem Melaminsynthesereaktor zugeführt wird.

4. Verfahren nach mindestens einem der Ansprüche 1 bis 3, **dadurch gekennzeichnet, dass** die Reinigung der Offgase bei einem Druck von 50 bis 600 bar durchgeführt wird.

5. Verfahren nach mindestens einem der Ansprüche 1 bis 4, **dadurch gekennzeichnet, dass** die Reinigung der Offgase in der ersten Stufe bei einer Temperatur von 160 bis 320 °C durchgeführt wird.

6. Verfahren nach mindestens einem der Ansprüche 1 bis 5, **dadurch gekennzeichnet, dass** die Reinigung der Offgase in der zweiten Stufe bei einer Temperatur von 135 bis 300 °C durchgeführt wird.

7. Verfahren nach mindestens einem der Ansprüche 1 bis 6, **dadurch gekennzeichnet, dass** die erste Stufe einen internen oder externen Wärmetauscher zur Energieabfuhr beinhaltet.

8. Verfahren nach mindestens einem der Ansprüche 1 bis 7, **dadurch gekennzeichnet, dass** den Offgasen Kohlendioxid zugesetzt wird.

9. Verfahren nach mindestens einem der Ansprüche 1 bis 8, **dadurch gekennzeichnet, dass** die Offgase über einen Offgasverteiler oberhalb oder unterhalb des Flüssigkeitsspiegels der melaminvorprodukt- und NH₃-hältigen Kreislaufharnstoffschmelze der ersten Stufe eingebracht werden.

10. Verfahren nach mindestens einem der Ansprüche 1 bis 9 , **dadurch gekennzeichnet dass** die frische Harnstoffschmelze in der zweiten Stufe auf mehrere Teilströme aufgeteilt und in unterschiedlicher Höhe eingebracht wird.

11. Verfahren zur Herstellung von Melamin, **dadurch gekennzeichnet, dass** dem Melaminsynthesereaktor eine Mischung aus frischer Harnstoffschmelze und melaminvorprodukt- und NH₃-hältiger Kreislaufharnstoffschmelze zugeführt wird.

12. Gaswäscher, insbesondere Offgaswäscher zur Reinigung verunreinigter Gase, insbesondere zur Durchführung des Verfahrens nach Anspruch 1 mit zwei übereinander angeordneten Stufen (11,12), wobei im oberen Abschnitt der unteren Stufe ein Sprühturm (13) und im unteren Abschnitt ein Gasverteiler (14) und im oberen Teil der oberen Stufe (11) ein Sprühturm (15) und mit einem oder mehreren, dem Sprühturm gegenüber angeordneten Siebböden (16), wobei die gereinigten Gase am Kopf des Gaswäschers und die Schmelz am Sumpf der unteren Stufe entnommen werden.

13. Gaswäscher nach Anspruch 12 mit einer weiteren Gaszuführung im unteren Teil der unteren Stufe.

14. Gaswäscher nach Anspruch 12 oder 13 mit einem Heizmantel.

15. Gasverteiler für die Zuführung von Gasen in eine Schmelze mit einem Zentralrohr (19), das von einem Heizmantel (21) umgeben ist, der durch ein thermisches Isoliermittel von der Schmelze getrennt ist, insbesondere durch einen gasgespülten Ringraum, mit Düsenbohrungen für den Gasaustritt.

## Claims

1. Process for purifying off-gases from a highpressure melamine plant with formation of melamine precursors, **characterized in that**
- the off-gases are brought into contact in a first stage with melamine-precursor-containing and NH₃-containing recirculated urea melt and
- in a second stage with fresh urea melt.

2. Process according to Claim 1, **characterized in that** the melamine-precursor-containing and NH₃-containing recirculated urea melt is a mixture of urea melt from the second stage and urea melt recirculated via the circuit to the first stage.

3. Process according to Claim 1 or 2, **characterized in that** a portion of the melamine-precursor-containing and NH₃-containing recirculated urea melt is fed to the melamine synthesis reactor.

4. Process according to at least one of Claims 1 to 3, **characterized in that** the off-gases are purified at a pressure of from 50 to 600 bar.

5. Process according to at least one of Claims 1 to 4, **characterized in that** the off-gases are purified in the first stage at a temperature of from 160 to 320°C.

6. Process according to at least one of Claims 1 to 5, **characterized in that** the off-gases are purified in the second stage at a temperature of from 135 to 300°C.

7. Process according to at least one of Claims 1 to 6, **characterized in that** the first stage comprises an internal or external heat exchanger for energy removal.

8. Process according to at least one of Claims 1 to 7, **characterized in that** carbon dioxide is added to the off-gases.

9. Process according to at least one of Claims 1 to 8, **characterized in that** the off-gases are introduced via an off-gas distributor above or below the liquid surface of the melamine-precursor-containing and NH₃-containing recirculated urea melt of the first stage.

10. Process according to at least one of Claims 1 to 9, **characterized in that** the fresh urea melt is divided in the second stage into a plurality of substreams and introduced at different heights.

11. Process for preparing melamine, **characterized in that** a mixture of fresh urea melt and melamine-precursor-containing and NH₃-containing recirculated urea melt is fed to the melamine synthesis reactor.

12. Gas scrubber, in particular off-gas scrubber for purifying contaminated gases, in particular for carrying out the process according to Claim 1 having two stages (11, 12) disposed one above the other, where in the upper section of the lower stage a spraying tower (13) and in the lower section a gas distributor (14) and in the upper section of the upper stage (11) a spraying tower (15) and having one or more sieve trays (16) disposed opposite the spraying tower, the purified gases being taken off at the top of the gas scrubber and the melt being take off at the bottom of the lower stage.

13. Gas scrubber according to Claim 12 having a further gas feed in the lower part of the lower stage.

14. Gas Scrubber according to Claim 12 or 13 having a heating jacket.

15. Gas distributor for feeding gases into the melt having a central tube (19) which is surronded by the heating jacket (21) which is seperated from the melt by a thermal insulating medium, in particular by a gas-purged annular space, having nozzle bore holes for the gas exit.

## Revendications

1. Procédé en vue de la purification de gaz de rejet provenant d'une installation de mélamine haute pression avec formation de produits précurseurs de mélamine,
**caractérisé**
- **en ce que** les gaz de rejet sont mis en contact dans un premier étage avec un bain d'urée en fusion en circuit fermé, contenant un produit précurseur de mélamine et du NH₃ et,
- dans un deuxième étage, avec un bain d'urée en fusion frais.

2. Procédé selon la revendication 1, **caractérisé en ce que** le bain d'urée en fusion en circuit fermé contenant un produit précurseur de mélamine et du NH₃ est un mélange du bain d'urée en fusion provenant du deuxième étage et du bain d'urée en fusion reconduit dans le premier étage par l'intermédiaire du circuit fermé.

3. Procédé selon la revendication 1 ou 2, **caractérisé en ce qu'**une partie du bain d'urée en fusion en circuit fermé, contenant un produit précurseur de mélamine et du NH₃, est acheminée au réacteur de synthèse de mélamine.

4. Procédé selon au moins une des revendications 1 à 3, **caractérisé en ce que** la purification des gaz de rejet est effectuée à une pression de 50 à 600 bars.

5. Procédé selon au moins une des revendications 1 à 4, **caractérisé en ce que** la purification des gaz de rejet dans le premier étage est effectuée à une température de 160 à 320°C.

6. Procédé selon au moins une des revendications 1 à 5, **caractérisé en ce que** la purification des gaz de rejet dans le deuxième étage est effectuée à une température de 135 à 300°C.

7. Procédé selon au moins une des revendications 1 à 6, **caractérisé en ce que** le premier étage contient un échangeur thermique interne ou externe en vue de l'évacuation de l'énergie.

8. Procédé selon au moins une des revendications 1 à 7, **caractérisé en ce que** l'on ajoute du dioxyde de carbone aux gaz de rejet.

9. Procédé selon au moins une des revendications 1 à 8, **caractérisé en ce que** les gaz de rejet sont introduits par l'intermédiaire d'une tubulure de distribution des gaz de rejet au-dessus ou en-dessous du niveau du liquide du bain d'urée en fusion en circuit fermé, contenant un produit précurseur de mélamine et du NH₃ du premier étage.

10. Procédé selon au moins une des revendications 1 à 9, **caractérisé en ce que** le bain d'urée en fusion frais est subdivisé dans le deuxième étage en plusieurs courants partiels et est introduit à des hauteurs différentes.

11. Procédé en vue de la fabrication de mélamine, **caractérisé en ce que** l'on achemine au réacteur de synthèse de mélamine un mélange de bain d'urée en fusion frais et de bain d'urée en fusion en circuit fermé, contenant un produit précurseur de mélamine et du NH₃.

12. Épurateur de gaz, en particulier, épurateur de gaz de rejet, en vue de la purification de gaz contaminés, en particulier en vue de l'exécution du procédé selon la revendication 1, avec deux étages disposés l'un au-dessus de l'autre (11, 12), une tour de pulvérisation (13) étant disposée dans la section supérieure de l'étage inférieur et une tubulure de distribution de gaz (14) étant disposée dans la section inférieure et une tour de pulvérisation (15) et un ou plusieurs fonds de tamis (16), disposés en face de la tour de pulvérisation, étant disposés dans la partie supérieure de l'étage supérieur (11), les gaz purifiés étant prélevés à la tête de l'épurateur de gaz et le bain en fusion l'étant au puits de l'étage inférieur.

13. Épurateur selon la revendication 12 ayant une amenée de gaz supplémentaire dans la partie inférieure de l'étage inférieur.

14. Épurateur de gaz selon la revendication 12 ou 13 ayant une chemise de chauffage.

15. Distributeur de gaz en vue de l'alimentation de gaz dans un bain en fusion avec un tube central (19), qui est entouré d'une chemise de chauffage (21), qui est séparée du bain en fusion par un milieu d'isolation thermique, en particulier par un espace annulaire purgé aux gaz, ayant des alésages de buse pour la sortie du gaz.
